# EUROPEAN PATENT APPLICATION

(11) **EP 1 790 359 A1**
(43) Date of publication of application: **30.05.2007**
(21) Application number: 06380311.8
(22) Date of filing: 24.11.2006
(51) Int. Cl.: A61L 9/03, A01M 1/20

(54) **Evaporator for liquids with an adjustable heater**

(30) Priority: 24.11.2005 ES 200502582
(71) Applicant: Igual Alonso, Jose Luis, 03007 Alicante (ES)
(72) Inventor: Igual Alonso, Jose Luis, 03007 Alicante (ES)
(74) Representative: Gonzalez Gomez, Maria Virtudes

(57) **Abstract**

Adjustable heather/evaporator for liquids of the type that includes a set of heating elements (1) that can rise the temperature of an air freshener or insecticide product, contained in a bottle with a wick through which the product evaporates, in which there are means (6,7,8) to supply two levels of heat to said heating elements (1) depending on the product to be evaporated.

## Description

### PURPOSE OF THE INVENTION

This invention refers to a heater/evaporator for liquids, of the type normally used to supply air fresheners and insecticides and which include an electrical element to heat the product concerned, contained in a recipient, to facilitate its evaporation.

The object of the invention is to provide an adjustable heater/evaporator specifically designed to provide two different temperatures according to the nature of the product to be dispensed.

### BACKGROUND TO THE INVENTION

The wide range of systems for supplying an air freshener or insecticide to the atmosphere includes a known system that consists of the use of a bottle containing the product concerned, with a wick. The bottle is fitted in a detachable manner into the casing of the device in a position in which the wick is close to a heating element or elements such that the increased heat considerably aids the wick to evaporate the air freshener as it is absorbed by the wick from the bottom of the bottle.

The heating capacity of this element or set of elements is fixed, that is, pre-set, so that for the optimal functioning of the heater/evaporator, two types of apparatus need to be made, one for air fresheners and the other for insecticides, since the latter require a higher temperature, of up to 110° C, compared to temperatures of under 80° C needed for air fresheners.

This temperature difference is related to the types of solvents and alcohols used in their manufacture and means that a device that works correctly with air fresheners is not the most suitable for insecticides, and *vice versa.*

### DESCRIPTION OF THE INVENTION

The heater/evaporator which the invention proposes solves the problem described above in a fully satisfactory way so that it is equally suitable for either of the aforementioned products, since it is capable of providing both working temperatures, either at the user's choice or automatically, as described below.

For this, and more specifically, the device disclosed is based on the use of at least two heating elements located next to the opening through which the wick passes as it emerges from the bottle. Its features centre on the use of a diode at the input to the power supply circuit to these elements which can be short-circuited so that the elements can supply enough heat for the device's working temperature to reach the maximum, for example, close to 110° C for insecticides when the diode is short-circuited and not operating, while when the diode is operating it causes a voltage drop in the power supply that lowers the effective power of the elements to the other pre-set level, that close to the 80 °C required by air fresheners.

As stated, this adjustment to the effective power of the heating elements can be made manually and on purpose by means of the relevant micro switch, directly or indirectly accessible, or automatically in such a way that using two different types of bottles for the products, one of which has a protuberance which the other lacks, the micro switch is activated or not when the bottle is inserted, thus short-circuiting the voltage reduction diode or not.

This basic structure makes it feasible for the device to use two, three or four heating elements and the diode that adjusts the working voltage of those elements may in turn be materialised as another element which carries out the same function as the diode, with the advantage that it can control the temperature of the other elements.

### DESCRIPTION OF DRAWINGS

To complement this description and to help with the better understanding of the features of the invention, according to a preferred example of practical embodiment thereof, this description is accompanied, as an integral part of it, by a set of drawings that illustrate but do not restrict it, showing:
Figure 1 shows a plan view of the printed circuit board for a heater/evaporator made according to the object of this invention on which the heating elements are installed together with the opening through which the bottle supplying the product passes.
Figure 2 shows a side elevation view of the two types of bottle designed for the automatic adjustment of the voltage in the device according to the type of product to be supplied to the atmosphere.

### PREFERRED EMBODIMENT OF THE INVENTION

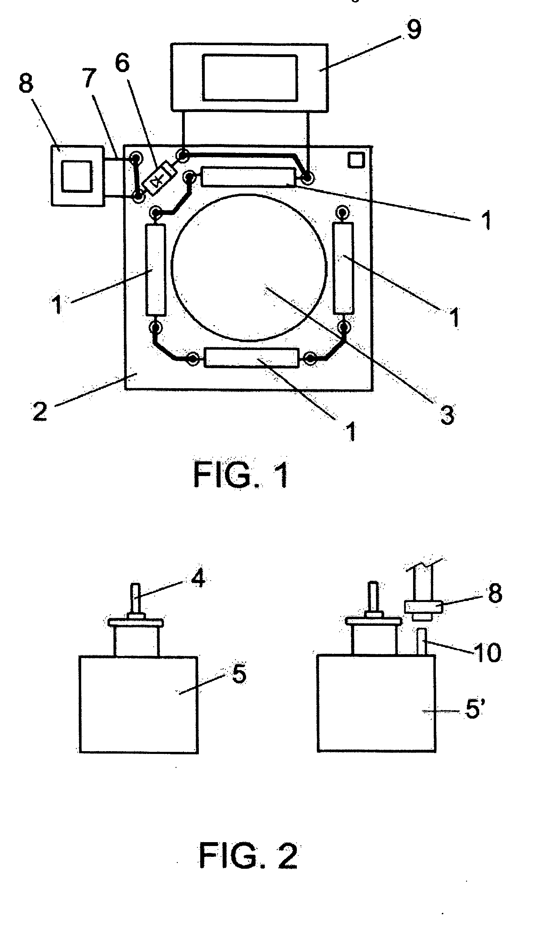

In the example of practical embodiment chosen and as shown in Figure 1, four heating elements (1) are used, arranged at equal angles on the printed circuit board (2) which supports them, surrounding the opening (3) in the board (2) through which the wick (4) passes that extracts the product concerned from its bottle (5) fitted in the casing of the heater/evaporator, but clearly the number of elements (3) may vary, as stated previously, using only three or two elements and the number of elements may even vary positively, that is, using more than four.

According to the invention, the power supply circuit for the elements (1) includes a diode (6) short-circuited with a by-pass (7), fitted with a micro-switch (8) so that when the micro-switch (8) is closed, the device's power supply voltage, supplied through the classic master switch (9), passes through this by-pass (7) and the diode (6) remains inoperative, while when the micro-switch (8) is open, the diode (6) becomes operative in the elements' (1) power supply circuit, causing a voltage drop in the power supply to them that results in a loss of power in them and, therefore, the reduced heating of the wick (4).

The micro-switch (8) may be operated manually but will preferably be automatic, specifically through the use of the two types of bottle (5-5') shown in Figure 2, so that the bottle (5) does not operate the micro-switch (8) when inserted into the device, it containing, for example, air freshener, while the bottles (5') intended for containing insecticides include a protuberance (10) which operates the micro-switch (8) when the bottle is inserted, changing the state in the elements' (1) power supply circuit.

It only remains to be noted that, as stated above, the diode (6) could be replaced with another element that can generate a regulated voltage drop.

## Claims

1. Adjustable heater/evaporator for liquids of the type that includes a set of heating elements that can raise the temperature of an air freshener or insecticide product, contained in a bottle with a wick through which the product evaporates, in which there are means to supply two levels of heat to these heating elements depending on whether the heater/evaporator is used for air fresheners or insecticides, to adjust the working temperature to the different requirements of these two types of products.

2. Adjustable heater/evaporator for liquids, according to claim 1, **characterised in that** these means of varying the heating power of the elements consists of a diode in the power supply circuit to these elements, equipped with a by-pass containing a micro-switch so that when the micro-switch is open the diode is operative while when the micro-switch is closed it is inoperative, causing a voltage drop or otherwise in the power supply to the elements.

3. Adjustable heater/evaporator for liquids, according to the preceding claims, **characterised in that** this diode may optionally be replaced with a complementary element that can also be short-circuited by the micro-switch.

4. Adjustable heater/evaporator for liquids, according to the preceding claims, **characterised in that** two types of bottles are used in it, one for air fresheners and the other for insecticides, one type of bottle having a protuberance which, when the bottle is inserted, operates the micro-switch to short-circuit the diode so that it is the bottle itself that determines the heating power supplied by the device when the bottle is inserted into it.
